# EUROPEAN PATENT APPLICATION

(11) **EP 4 715 037 A1**
(43) Date of publication of application: **25.03.2026**
(21) Application number: 24202021.2
(22) Date of filing: 23.09.2024
(51) Int. Cl.: C12M 1/34, B01F 25/431, B01L 3/00, G01N 27/28

(54) **MEASURING CELL UNIT FOR BIOPHARMA PROCESS SYSTEM, BIOPHARMA PROCESS SYSTEM, AND METHOD FOR EXCHANGING FLUIDS IN BIOPHARMA PROCESS SYSTEM**

(71) Applicant: Sartorius Stedim Biotech GmbH, 37079 Göttingen (DE)
(72) Inventor: REGEN, Thomas, 37079 Göttingen (DE); LUDOLPH, Niclas, 37079 Göttingen (DE); DÜCKER, Eibe, 37079 Göttingen (DE)
(74) Representative: Prinz & Partner mbB

(57) **Abstract**

A measuring cell unit (16) for a bioprocess system is shown, in particular for a biopharma process, comprising a flow pipe unit (28) with a first connection end (30) having an inlet channel (34), a second connection end (32) having an outlet channel (36) and a measuring chamber (38) between the inlet and the outlet channels (34, 36). The first and second connection ends (30, 32) are configured to connect the measuring cell unit (16) into the bioprocess system. Together, the inlet channel (34), the measuring chamber (38) and the outlet channel (36) define portions of a flow duct (40) through the measuring cell unit (16). Further, the flow pipe unit (16) comprises a sideward sensor insert hole (42) opening into the measuring chamber (38) such that a measuring sensor (26) can be inserted. The flow duct (40) further comprises a transitional portion (44) along which a cross-sectional area of the flow duct (40) increases from the inlet channel (34) towards the measuring chamber (38). Additionally, the measuring cell unit (16) comprises at least one flow deflection arm (46) extending into the transitional portion (44) and deflecting a fluid flowing through the flow duct (40).

Further, a bioprocess system and a method for displacing a first fluid by a second fluid are shown.

## Description

The invention relates to a measuring cell unit for a bioprocess system, in particular to a biopharma process system. Further, the invention relates to a bioprocess system, in particular a biopharma process system, comprising the measuring cell unit. Additionally, the invention relates to a method for exchanging fluids in the bioprocess system comprising the measuring cell unit.

In bioprocesses and subsequent purification steps, that is to say in upstream as well as in downstream steps of the bioprocess, the medium usually consists of a homogenous mixture comprising multiple substances mixed together. The mixture may change over time, in particular in regards to the concentration of specific substances, as further substances may be added throughout the bioprocess. Due to the addition of further substances such as buffer fluids, nutrients or similar, the medium may get separated into its different substances due to different densities as well as geometric restrictions of the bioprocess system. However, in order to obtain a meaningful measurement result and to be able to determine the analyte of interest, the medium needs to be a homogenous mixture.

In particular, when flow velocities are slow and the cross-sectional area at a measuring point or upstream of a measuring point widens, the substances are not or at least not homogenously mixed when one fluid is reaching the other fluid. Consequently, the obtained measurements may deviate from the true value and the response time of the measurement increases.

In other words: as different fluids may flow through the measuring cell, analysis of the fluid being present in the measuring cell unit is difficult at those moments in which a second fluid, reaching the measuring cell unit after the first fluid, must completely replace the first fluid within the measuring cell unit.

Therefore, it is an objective of the invention to provide a measuring cell unit for a bioprocess system that ensures a homogenous mixture of substances at a measuring point and a fast and complete replacement of the fluid presently filling the measuring cell unit by the newly pouring in fluid.

According to the invention this objective is achieved by a measuring cell unit for a bioprocess system, in particular for a biopharma process, comprising a flow pipe unit with a first connection end having an inlet channel, a second connection end having an outlet channel and a measuring chamber between the inlet and the outlet channels. The first and second connection ends are configured to connect the measuring cell unit into the bioprocess system. Together the inlet channel, the measuring chamber and the outlet channel define portions of a flow duct through the measuring cell unit. Further, the flow pipe unit comprises a sideward sensor insert hole opening into the measuring chamber such that a measuring sensor can be inserted. The flow duct further comprises a transitional portion along which a cross-sectional area of the flow duct increases from the inlet channel towards the measuring chamber. Additionally, the measuring cell unit comprises at least one flow deflection arm extending into the transitional portion and deflecting a fluid flowing through the flow duct.

In other words, the idea is to have the flow deflection arm or the flow deflection arms positioned upstream of the measuring chamber, i.e. upstream of the measuring sensors, such that the second fluid covers the whole cross-sectional area of the measuring chamber upstream of the measuring sensor to displace the old medium, i.e. the first fluid is quickly and effectively displaced by the second fluid. However, it is important to note that the flow deflection arm/arms are not intended to work as a static mixer but instead are supposed to evenly distribute the flow of the second fluid in order to effectively replace the first, i.e. old, fluid.

Put differently, the measuring cell unit is based on the principal that by including one or more flow deflection arms the fluid flow is partly deflected even in those parts of the measuring chamber in which the first fluid would hardly be displaced without such deflection arms as this fluid is in a low-flow zone. In turn, the deflection of the fluid flow reduces the response time of the measuring sensors, such that the time required to obtain the meaningful measurement is reduced.

The flow pipe's inlet and outlet preferably have a circular cross-section and may have any diameter larger than 1/8" (3.175 mm). Therefore, the measuring cell unit may be used for nearly any cross-sectional area.

Depending on where the measuring cell unit is placed in the bioprocess system, the measuring chamber can include an additional inlet for introducing a further substance such as gas or buffer fluid. Such an additional inlet is particularly helpful for measuring cell units having a long flow duct.

The measuring cell unit may be used in any bioprocess system requiring a slow flow rate, in the order of a few mm/min, and/or the introduction of further substances. For example, the measuring cell unit can be used in bioprocesses with buffer exchange such as chromatography or virus inactivation.

According to a preferred embodiment the at least one flow arm and/or the flow pipe unit are made from a corrosion resistant material, preferably plastic, such that the flow deflection arm and/or the flow pipe unit are single-use parts.

Furthermore, using plastic, e.g. polyamide 12 (PA12), the components are easy to produce and production costs can be kept at a minimum, for example by using additive manufacturing, e.g. 3D-printing. This also allows producing the parts in a variety of sizes without having a lot of limitations.

Preferably, the at least one flow deflection arm is plate shaped and/or extends to opposite sides of the transitional portion. Therefore, the flow deflection arm is positioned upstream of the measuring chamber ensuring that the fluid is homogeneously distributed over the cross-section when it passes any measurement sensor located in the measuring chamber.

According to an embodiment of the invention, the at least one deflection arm is perpendicular to an axial direction of the transitional portion. Put differently, the flow deflection arm splits the flow duct at least in half such that a relative movement is introduced into the fluid flow.

Generally, the deflection arm may have one fixed and an opposite protruding, free end or both ends may be fixed to the wall delimiting the flow duct.

According to an embodiment, flow deflection arms are provided forming a grid, in particular wherein the flow deflection arms are arranged perpendicular to each other. The flow deflection arms comprise first flow deflection arms and second flow deflection arms which are arranged perpendicular to each other as well as perpendicular to the axial direction of the transitional portion. Additionally, the first flow deflection arms may be parallel to each other while the second flow deflection arms are parallel to each other as well. That way more relative movement can be introduced into the fluid flow such that the previous medium is displaced more efficiently.

According to an alternative embodiment, the flow deflecting arms together form a propeller like static structure generating a swirl flow. Due to the swirl flow the new fluid can be urged in low-flow zones and the previous medium is displaced such that the response time for a significant measurement result is reduced.

The measuring chamber may have a cross-section with a dimension larger than that of the transitional area. Accordingly, the at least one deflection arm may be inclined towards those areas most distanced from the centre of the cross-section, i.e. typical low-flow zones. In particular, at least one flow deflection arm is angled to a bottom portion and at least one flow deflection arm is angled to an opposite roof portion of the measuring chamber. Put differently, the at least one flow deflection arm is inclined with respect to the axial direction of the transitional portion such that the relative movement of the new fluid is directed towards the areas of the fluid duct that extend past the cross-sectional area of the transitional portion.

According to a further embodiment the at least one flow deflection arm is integral with an inner wall of the measuring chamber delimiting the flow duct. For example, the at least one flow deflecting arm is integral with the flow pipe unit. This reduces the number of parts required to produce the measuring cell unit, therefore decreasing the complexity of the manufacturing process.

According to a further embodiment, an insert part is provided which is inserted into the interior of the flow pipe unit and limits the measuring chamber in the flow direction in the upstream direction. The insert part defines an intermediate portion between the transitional portion and the measuring chamber, wherein the flow duct portion defined by the insert part is merging with the adjacent downstream wall of the measuring chamber and/or the wall defining the transitional part without defining a shoulder and/or with the same curvature. In other words, the insert part provides a continuous transition from the cross-sectional area of the transitional portion to the cross-sectional area of the measuring chamber. For this purpose, the insert part may for example be wedge-shaped such that the cross-sectional area gradually increases without introducing any kinks that would cause unwanted movements within the fluid flow.

According to an alternative embodiment, an insert part is provided which is inserted into the interior of the flow pipe unit and in flow direction limits the measuring chamber in the upstream direction. The insert part therefore defines an intermediate portion between the transitional portion and the measuring chamber. Further, the at least one flow deflecting arm is an integral portion of the insert part. By forming the at least one flow deflection arm integrally with the insert part, it is possible to provide a modular measuring cell unit, such that depending on the specific needs of a certain application of the measuring cell unit, i.e. based on the specific bioprocess, the insert part may be chosen and inserted into the interior of the flow pipe unit. Consequently, an overall cost reduction may be achieved, as a variety of inserts may be obtained separately from the flow pipe unit which may be used for a multitude of bio processes.

The insert part comprising the at least one flow deflection arm may also be designed to gradually change from the transitional portion to the measuring chamber as explained before.

According to an embodiment, the insert part has sideward recesses for opposing sensors surfaces. This allows the insert part to be positioned directly upstream of the measuring sensor, simplifying the insertion process of the insert part and reducing the space required in the measuring chamber.

The measuring cell unit may comprise at least one measuring device with at least one sensor is provided, the at least one sensor protruding into the measuring chamber via an associated sensor insert hole. Thus, the sensor or sensors are part of the measuring cell unit.

The objective is also achieved by a bioprocess system, in particular for a biopharma process, comprising a container, preferably a bioreactor, containing a liquid, at least one tube system, at least one measuring cell unit as previously described, and at least one measuring device with at least one sensor. The sensor of the measuring device is positioned such that it is protruding into the measuring chamber via the sensor insert hole.

The measuring cell unit may be inserted at any point in the bioprocess system where a measurement is required. Possible measurements include the pH-level, the temperature, the pressure, the flow rate, the oxygen content, the conductivity, the viscosity, and optical parameters.

In order to provide a stable connection between the measuring cell unit and the tube system of the bioprocess system, the measuring cell unit is connected to the further components of the bioprocess system via hose barbs that ensure a durable and sealed connection.

According to an embodiment, the entire bioprocess system, but at least all wetted components of the bioprocess system, are single-use parts. This makes using the bioprocess system much easier as the entire bioprocess system can be disposed afterwards as a whole. Otherwise, it would be necessary to provide sterile connections between single-use and reusable components and to separate them before disposing the single-use components. Therefore, by having the entire bioprocess system consisting of single-use components, the required time for preparation and deconstruction can be reduced.

The objective is also achieved by a method for exchanging fluids in a bioprocess system, in particular wherein a first fluid flowing through the flow duct is to be replaced by a second fluid. The method includes the following steps,
- inserting the second fluid into the bioprocess system upstream of the measuring cell unit,
- deflecting the second fluid downstream of the inlet channel and upstream of the measuring chamber by at least one flow deflection arm such that the second fluid fills the cross-sectional area of the measuring chamber, and
- displacing the first fluid in the measuring chamber by the inserted second fluid.

In other words, the steps of the bioprocess do not need to be changed, as the at least one flow deflection arm of the measuring cell unit ensures complete and quick displacement of the first fluid.

Further features and advantages of the invention will become apparent from the following description and from the accompanying drawings to which reference is made. In the drawings,
- Figure 1 shows a schematic view of a bioprocess system according to the invention;
- Figure 2 shows a sectional view of a first embodiment of a measuring cell unit according to the invention inserted into the bioprocess system shown in Figure 1;
- Figure 3 shows an insert part placed in the measuring cell unit shown in Figure 2;
- Figure 4 shows a second embodiment of the measuring cell unit according to the invention, and;
- Figures 5A to 5D show perspective views of insert parts that may be used in the measuring cell unit shown in figure 4, wherein Figure 5A shows a first version of the insert part, Figure 5B shows a second version of the insert part, Figure 5C shows a third version of the insert part, and Figure 5D shows a forth version of the insert part.

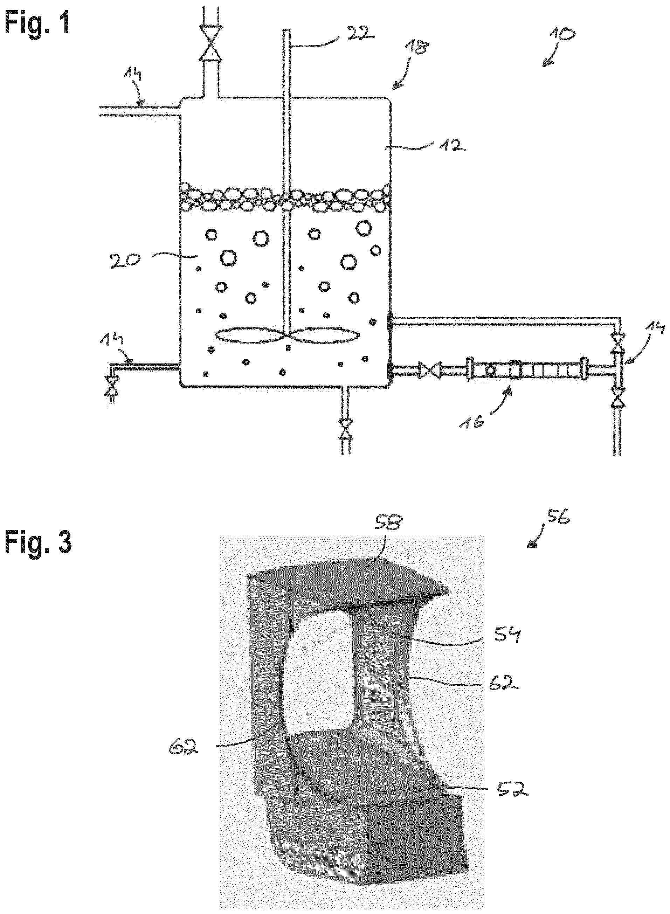

Figure 1 shows a bioprocess system 10 with a container 12, a tube system 14, and a measuring cell unit 16.

In the embodiment shown, the bioprocess system 10 is a biopharma process system such that the container 12 corresponds to a bioreactor 18. In particular, in the biopharma process system a bioprocess with a buffer exchange, for example chromatography or virus inactivation, is performed in a controlled manner.

The bioreactor 18 contains a medium 20 consisting of a mixture of substances. In order to ensure that the medium 20 is a homogeneous mixture, the bioreactor 18 further comprises a mixer 22 which continuously mixes the medium 20 contained in the bioreactor 18.

The bioreactor 18 is connected to the tube system 14 on its upstream and downstream sides such that new substances, e.g. buffer fluid or gas, may be added to the mixture and the mixture may be removed from the bioreactor 18.

In the embodiment shown in Figure 1, the measuring cell unit 16 is positioned downstream of the bioreactor 18 and inserted into the tube system 14. Alternatively, the measuring cell unit 16 may also be placed upstream of the bioreactor 18 or any other place where a measurement is required and/or desired.

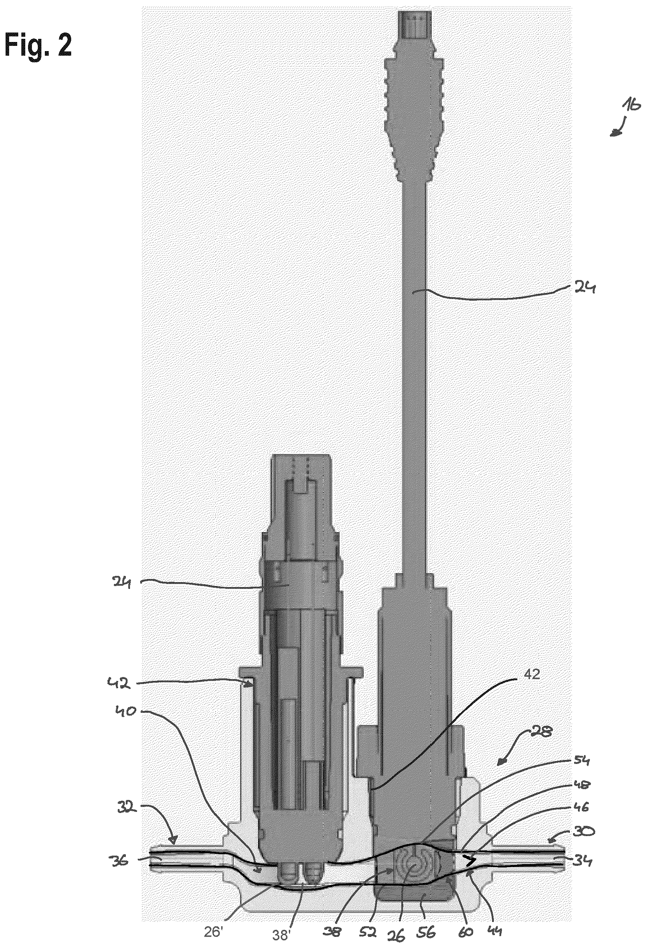

The measuring cell unit 16 is used for introducing measuring devices 24 (shown in Figure 2) into the fluid stream leaving or entering the bioreactor 18.

Each measuring device 24 comprises at least one sensor 26 for detecting or measuring parameters of the medium 20 which are characteristic for the biological reaction and/or the medium 20, in particular an analyte of interest, in the measuring cell unit 16. Depending on the reactants, the biological reaction and other circumstances, sensors 26 of the following types may be introduced into the measuring cell unit 16: pH sensor (e.g. an optochemical or electrochemical pH sensor), dissolved oxygen sensor, dissolved carbon dioxide sensor, temperature sensor, viscosity sensor, microscope, fiber Bragg sensor, conductivity sensor, capacitance sensor, refractometer, flow sensor, pressure sensor, optical sensor (e.g. UV, Raman, NIR, fluorescence), exhaust gas analyser (e.g. IR spectroscope), and image sensor (e.g. hyperspectral imager, holographic phase imager). The above list is not exhaustive.

The measuring cell unit 16 is inserted into the bioprocess system 10 and connected to the other components upstream and downstream using hose barbs. In the embodiment shown here, the measuring cell unit 16 is connected on both sides to the tube system 14.

Figure 2 shows the measuring cell unit 16 in more detail. Since the measuring cell unit 16 is essentially part of the tube system 14, the measuring cell unit 16 comprises a flow pipe unit 28 with a first connection end 30 and an opposite second connection end 32. Therefore, the measuring cell unit 16 is connected to the tube system 14 via its two connection ends 30, 32 which are fixed to the adjacent tube system 14 via the hose barbs.

The first connection end 30 of the measuring cell unit 16 defines an inlet channel 34, while the second connection end 32 defines an outlet channel 36. Together with a measuring chamber 38 positioned there between, the inlet channel 34 and the outlet channel 36 each form portions of a flow duct 40 that leads through the measuring cell unit 16.

The measuring cell unit 16, in particular the flow duct 40, has a circular cross-section at the inlet and outlet channels 34, 36 with a diameter corresponding e.g. to at least 1/8" (3.175 mm). The flow pipe unit 28 can be 3D-printed.

In order to allow measurements to be taken, the flow pipe unit 28 has at least one sideward sensor insert hole 42 such that when the measuring device 24 is inserted, the sensor 26 of the measuring device 24 is positioned inside the measuring chamber 38. The term "sideward" refers to the flow direction and the flow duct direction and is not related to vertical or horizontal directions.

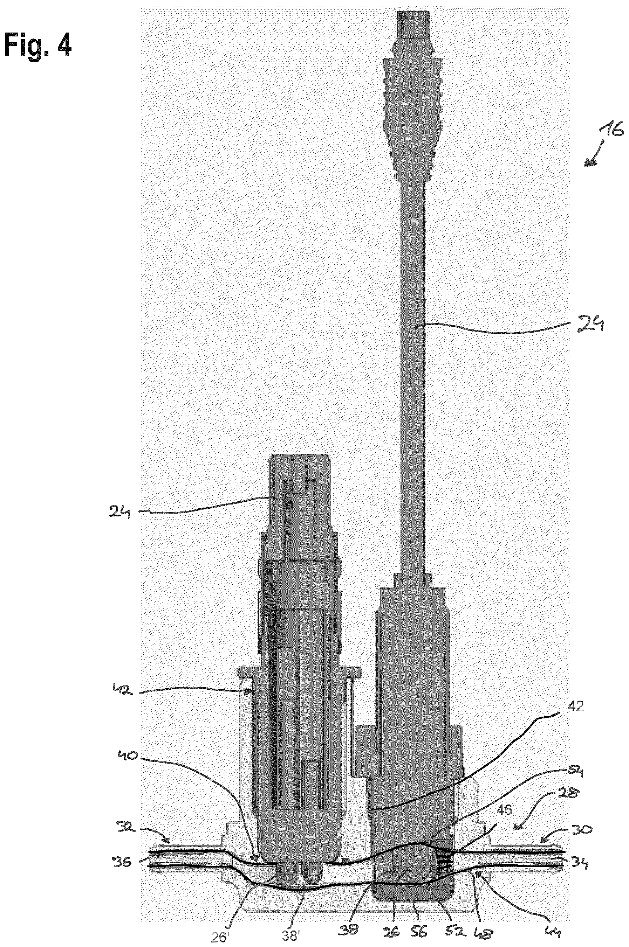

In the embodiments according to Figures 2 and 4, two sensors 26, 26' are arranged in flow direction behind each other. Sensor 26 is a conductivity sensor and sensor 26' a pH sensor. The measuring chambers 23 of sensor 26 has a larger cross sectional area than the measuring chamber 38' of downstream sensor 26'. Alternatively, the measuring chambers 28, 28' are merging with each other to define one larger measuring chamber 28.

As can be seen in Figure 2, the measuring chamber 38 has an increased cross-sectional area compared to the remaining flow duct 40. Accordingly, the cross-sectional area increases from the inlet channel 34 towards the measuring chamber 38 in a transitional portion 44 in which the cross-sectional area constantly increases, e.g. by having a conical shape.

As mentioned previously, the medium 20 contained in the bioreactor 18 is a liquid and a mixture of substances, which usually have different densities such that continued mixing is required to maintain a homogeneous mixture.

In particular when flow velocities are low, as is typical for bioprocesses which have a flow velocity in the order of a few mm/min, the substances contained in the medium 20 may start to separate from each other. Especially due to the widening of the cross-sectional area in the transitional portion 44, where the velocity further drops, substances with higher density may separate from substances with lower density such that no reliable measurement results can be obtained in that state.

Additionally, as the medium 20 of a bioprocess changes over time, it may be necessary to displace the medium 20 currently in the measuring cell unit 16 with the medium 20 freshly released from the bioreactor 18. As the medium compositions and its densities may differ as well, it is not possible to obtain significant results until the previous medium 20 is entirely displaced.

In order to accelerate displacement of the previous medium, the measuring cell unit 16 further comprises at least one flow deflection arm 46 which extends into the transitional portion 44 and deflects the fluid flowing through the flow duct 40.

In the embodiment shown in Figure 2, the flow deflection arms 46 are integrally formed with an inner wall 48 delimiting the flow duct 40 in the transitional portion 44, such that the flow deflection arm 46 is integral with the flow pipe unit 28.

The flow deflection arms 46 are inclined with respect to the centre of the cross-section of the transitional portion 44, such that the flow deflection arms 46 deflect the fluid flow towards those areas in the measuring chamber 38 that are most distanced from the centre of the cross-section. In other words, one of the flow deflection arms 46 is angled to a bottom portion 52 and the other flow deflection arm 46 is angled to an opposite roof portion 54 of the measuring chamber 38.

In the embodiment of the measuring cell unit 16 shown in Figure 2, the measuring cell unit 16 also comprises an insert part 56 that is inserted into the interior of the flow pipe unit 28.

As can be seen in Figure 3, the insert part 56 comprises a frame 58 which gradually widens. In the embodiment shown here, the frame 58 includes wedge-shaped parts causing the change in the cross-sectional area.

The insert part 56 defines an intermediate portion 60 between the transitional portion 44 and the measuring chamber 38 as the insert part 56 is positioned there between.

The insert part 56 is formed such that its cross-sectional area on the upstream side corresponds to the cross-sectional area of the downstream end of the transitional portion 44, while its cross-sectional area on the downstream side corresponds to the cross-sectional area of the wall within the flow pipe unit 28 at the measuring chamber 38. Accordingly, the insert part 56 merges with the adjacent downstream wall of the measuring chamber 38 and the adjacent upstream wall of the transitional portion 44 such that there is no shoulder or hard kink in the flow duct 40 and instead the cross-sectional area continuously widens.

The cross-sectional area of the insert part 56 gradually widens from the transitional portion 44 to the measuring chamber 38.

As can also be seen in Figures 2 and 3, the insert part 56 partly defines the bottom portion 52 and the roof portion 54 of the measuring chamber 38 and has sideward recesses 62. These sideward recesses 62 correspond to the form of opposing sensor surfaces, such that the insert part 56 can be placed directly upstream of the first measuring device 24.

Based on Figures 1 to 3 the effect of the measuring cell unit 16 in regards to obtaining meaningful measurement results in as short a time as possible will be described in the following.

When the medium 20 is released from the bioreactor 18 it flows through the tube system 14 towards the measuring cell unit 16. In the measuring cell unit 16 the required measurements are taken, using the measuring devices 24 inserted into the measuring chamber 38 having two opposed sensor surfaces arranged in or adjacent to the sideward recesses 62.

As a bioprocess is an ever changing process and substances are constantly added and/or react with substances contained in the medium 20, the medium 20 constantly changes and therefore measurements need to be taken throughout the entire time of the bioprocess.

The medium 20 inside the measuring cell unit 16 at the time of the first measurement will be referred to as the first fluid 64 in the following. Results regarding the analyte of interest of the first medium 20 can be taken without further problems.

After some time has passed and substances in the medium 20 have reacted with each other or a further substance and/or a different fluid has been added into the medium 20 upstream of the measuring cell unit 16 the measurements need to be taken again for what is referred to as the second fluid.

Once the second fluid reaches the measuring cell unit 16, in particular the transitional portion 44, i.e. is downstream of the inlet channel 34 and upstream of the measuring chamber 38, the second fluid is deflected towards the regions of the measuring chamber 38 furthest away from the center of the cross-sectional area by the flow deflection arms 46, such that the second fluid fills the entire cross-sectional area of the measuring chamber 38.

The at least one flow deflection arms 46 distributes the second fluid towards the sensor 26 of the measuring device 24 more evenly than without the deflection arm 46.

Due to the redirection of the fluid flow with the flow deflection arms 46 the first and second fluid mix and the first fluid as well as the mixture of the first and second fluid are effectively displaced by the continued insertion of the second fluid into the measuring cell unit 16.

Put differently, by deflecting the second fluid upstream of the measuring chamber 38 the first fluid can effectively be displaced through mixing with the second fluid.

The relative movement of the second fluid introduced by the flow deflection arms 46 enables a fast displacement of the first fluid such that the response time of the sensor 26 can be reduced.

As the cross sectional area of the downstream measuring chamber 38' is smaller than that of measuring chamber 38 the second fluid is also replacing the first fluid in the measuring chamber 38'.

The flow deflection arms and/or the flow pipe unit 28 are single-use components. Even more, all wetted parts of the bioprocess system 10 can be single-use components and hence made form plastic, preferably from Polyamid 12 (PA12).

According to a different embodiment of the measuring cell unit 16 (not shown in the Figures), the measuring cell unit 16 only comprises the flow deflection arms 46 and not the insert part 56. This reduces the production costs and fewer parts are required.

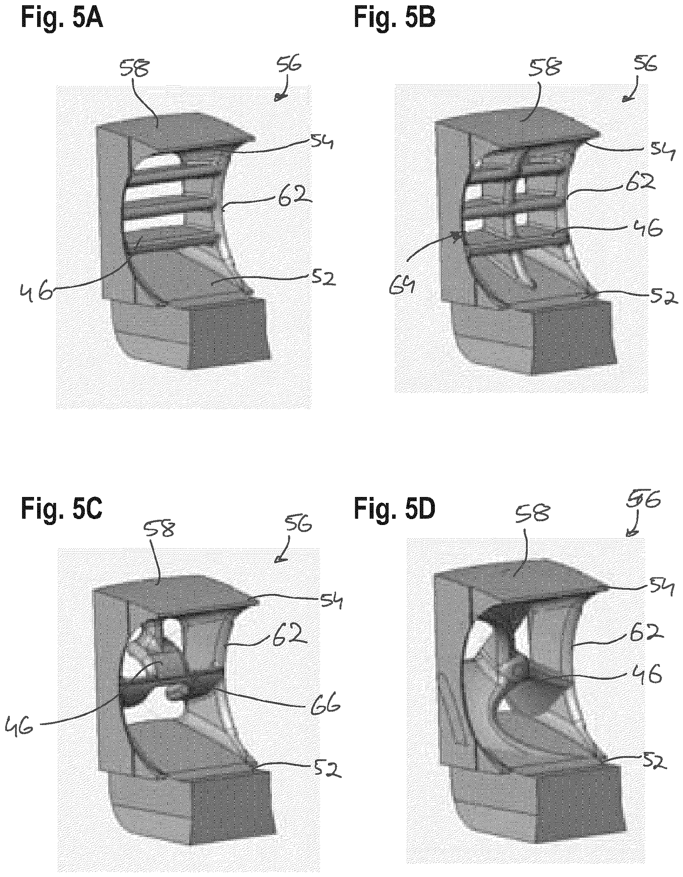

A further embodiment of the measuring cell unit 16 is shown in Figure 4. The difference with respect to the embodiment shown in Figure 2 is that the measuring cell unit 16 shown in Figure 4 does not comprise flow deflection arms 46 integrally formed with the inner wall 48 of the flow duct 40. Instead, the flow deflection arms 46 are integrally formed with the insert part 56, which is shown in more detail in Figures 5A to 5D.

Different arrangements of the flow deflection arms 46 are described based on different versions of the insert parts 56 shown in Figures 5A to 5D.

In the version of the insert part 56 shown in Figure 5A the flow deflection arms 46 are plate shaped and extend to opposite sides of the insert frame 58. As shown here, the plate shaped flow deflection arms 46 are arranged parallel to each other and simply split the fluid flow.

The insert part 56 shown in Figure 5A is inserted into the flow duct 40 immediately downstream of the transitional portion 44. The flow deflection arms 46 are perpendicular to an axial direction of the transitional portion 44 (which is the flow direction). The insert part 56 ensures the gradual increase in the cross-sectional area from the inlet channel 34 towards the measuring chamber 38 without defining a shoulder there between and with the same curvature at the border between these portions of the flow duct.

While in the embodiment shown in Figure 5A the plate shaped flow deflection arms 46 extend over the entire width of the insert part 56 and therefore the flow duct 40, it is also possible that the flow deflection arms 46 simply extend from one or more walls of the insert part 56 and end freely.

The insert part 56 shown in Figure 5B further comprises a second kind of flow deflection arm which is perpendicular to the flow deflection arms 46 that are arranged according to Figure 5A. That way, it is still ensured that the flow deflection arms 46 are perpendicular to an axial direction of the transitional portion 44 while forming a grid 64.

As shown in Figure 5B, the horizontal flow deflection arms 46 may be parallel to each other. Similarly, if the insert part 56 comprises multiple vertical flow deflection arms 46, the vertical flow deflection arms 46 may also be parallel to each other.

Figure 5C shows an insert part 56 with three bow-shaped deflection arms 46, two of which are inclined towards the roof portion 54 while one, central flow deflection arm 46 as inclined towards the bottom portion 52 of the measuring chamber 38.

In particular, the flow deflection arms 46 all extend from a common connection part 66 positioned in the centre of the insert part 56 and extending through the flow duct 40.

In Figure 5D, the flow deflection arms 46 are also integrally formed with the insert part 56 and form a propeller-like static structure which generates a swirl flow. That way mixing is achieved very effectively such that the first fluid may be displaced by the second fluid as fast as possible.

## Claims

1. A measuring cell unit (16) for a bioprocess system (10) comprising a flow pipe unit (28) with a first connection end (30) having an inlet channel (34), a second connection end (32) having an outlet channel (36) and a measuring chamber (38, 38') between the inlet and the outlet channels (34, 36), the first and second connection ends (30, 32) being configured to insert the measuring cell unit (16) into the bioprocess system (10),
the inlet channel (34), the measuring chamber (38) and the outlet channel (36) defining portions of a flow duct (40) through the measuring cell unit (16),
wherein the flow pipe unit (28) having a sideward sensor insert hole (42) opening into the measuring chamber (38) for inserting a measuring sensor (26),
wherein the flow duct (40) further comprising a transitional portion (44) along which a cross-sectional area of the flow duct (40) increases from the inlet channel (34) towards the measuring chamber (38),
the measuring cell unit (16) further comprising at least one flow deflection arm (46) extending into the transitional portion (44) and deflecting a fluid flow through the flow duct (40).

2. The measuring cell unit (16) according to claim 1, wherein the at least one flow deflection arm (46) and/or the flow pipe unit (28) are made from a corrosion resistant material, preferably plastic, such that the flow deflection arm (46) and/or the flow pipe unit (28) are single-use parts.

3. The measuring cell unit (16) according to claim 1 or 2, wherein the at least one flow deflection arm (46) is plate shaped and/or extends to opposite sides of the transitional portion (44).

4. The measuring cell unit (16) according to any of the preceding claims, wherein the at least one flow deflection arm (46) is perpendicular to an axial direction of the transitional portion (44).

5. The measuring cell unit (16) according to any of the preceding claims, wherein flow deflection arms (46) are provided forming a grid (64), in particular wherein flow deflection arms (46) are arranged perpendicular to each other.

6. The measuring cell unit (16) according to any of the preceding claims, wherein flow deflecting arms together form a propeller-like static structure generating a swirl flow.

7. The measuring cell unit (16) according to any of preceding claims, wherein the measuring chamber (38) has a cross-section with a dimension larger than that of the transitional area, wherein the at least one flow deflection arm (46) is inclined towards the area or areas most distanced from a center of the cross-section, in particular wherein at least one flow deflection arm (46) is angled to a bottom portion (52) and at least one flow deflection arm (46) is angled to an opposite roof portion (54) of the measuring chamber (38).

8. The measuring cell unit (16) according to any of the preceding claims, wherein the at least one flow deflection arm (46) is integral with an inner wall (48) delimiting the flow duct (40), in particular the at least one flow deflecting arm is integral with the flow pipe unit (28).

9. The measuring cell unit (16) according to any of the preceding claims, wherein an insert part (56) is provided which is inserted into the interior of the flow pipe unit (28) and, in flow direction, limits the measuring chamber (38) in the upstream direction, the insert part (56) defining an intermediate portion (60) between the transitional portion (44) and the measuring chamber (38), wherein the flow duct portion defined by the insert part (56) is merging with the adjacent downstream wall of the measuring chamber (38) and/or the wall defining the transitional part without defining a shoulder and/or with the same curvature.

10. The measuring cell unit (16) according to any of the preceding claims, wherein an insert part (56) is provided which is inserted into the interior of the flow pipe unit (28) and, in flow direction, limits the measuring chamber (38) in the upstream direction, the insert part (56) defining an intermediate portion (60) between the transitional portion (44) and the measuring chamber (38), the at least one flow deflecting arm is an integral portion of the insert part (56).

11. The measuring cell unit (16) according to claim 9 or 10, wherein the insert part (56) has opposite sideward recesses (62) for opposing sensor surfaces.

12. The measuring cell unit (16) according to any of the preceding claims, wherein at least one measuring device (24) with at least one sensor (26, 26') is provided, the at least one sensor (26, 26') protruding into the measuring chamber (38) via an associated sensor insert hole (42).

13. A bioprocess system (10), in particular a biopharma process system, comprising a container (12) containing a liquid, preferably a bioreactor (18), at least one tube system (14), at least one measuring cell unit (16) according to claim 12.

14. A method for exchanging fluids in a bioprocess system (10) according to claim 13, in particular in a measuring cell unit (16) according to any of claims 1 to 12, wherein a first fluid flowing through the flow duct (40) is replaced by a second fluid by the following steps:
- inserting the second fluid into the bioprocess system (10) upstream of the measuring cell unit (16),
- deflecting the second fluid downstream of the inlet channel (34) and upstream of the measuring chamber (38, 38') by at least one flow deflection arm (46) such that the second fluid fills the cross-sectional area of the measuring chamber (38, 38'), and
- displacing the first fluid in the measuring chamber (38, 38') by the inserted second fluid.
